# EUROPEAN PATENT SPECIFICATION

(11) **EP 3 137 057 B1**
(45) Date of publication and mention of the grant of the patent: **09.09.2020**
(21) Application number: 15786475.2
(22) Date of filing: 01.05.2015
(51) Int. Cl.: A61K 9/10, A61K 9/50, A61K 47/26, A61K 47/36, A61K 47/38

(54) **EXTENDED RELEASE LIQUID COMPOSITIONS OF METFORMIN**
PHARMAZEUTISCHE RETARD-ZUSAMMENSETZUNGEN VON METFORMIN
COMPOSITIONS DE METFORMINE LIQUIDES À LIBÉRATION PROLONGÉE

(30) Priority: 01.05.2014 IN 1182DE2014
(43) Date of publication of application: 08.03.2017
(73) Proprietor: Sun Pharmaceutical Industries Ltd, 400063 Mumbai, Maharashtra (IN)
(72) Inventor: KUMAR, Ashish, Jhajjar Haryana 124507 (IN); SHEAR, Rajesh Srikrishan, Gurgaon Haryana 122001 (IN); JAIN, SatishKumar, Bilaspur Chhattisgarh 495001 (IN); SINGH, Romi Barat, Varanasi Uttar Pradesh 221002 (IN); JAIN, Paras P., Amravati Maharashtra 444708 (IN)
(74) Representative: Cronin, Brian Harold John
(86) International application number: PCT/IB2015/053207
(87) International publication number: WO 2015/166472

(56) References cited:
- EP-B1- 1 140 027
- WO-A1-2004/012715
- WO-A1-2006/086856
- WO-A2-2010/045656
- FR-A1- 2 897 267
- US-A- 5 273 760
- US-A- 5 955 106
- US-A1- 2009 123 538
- US-A1- 2009 325 938
- US-A1- 2013 109 659
- LOPEZ-LIUCHI, JV ET AL.: 'Therapy for Type 2 Diabetes: Where Do We Stand After the UK Prospective Diabetes Study?' EUROPEAN JOURNAL OF ENDOCRINOLOGY, [Online] vol. 140, 1999, pages 4 - 6, XP055232351 Retrieved from the Internet: <URL:http://eje-online.org/conten/140/1/4.l ong> [retrieved on 2015-07-14]

## Description

### Field of the Invention

The present invention relates to extended release liquid compositions of metformin. The extended release liquid compositions are in the form of suspensions or reconstituted powder for suspensions. Said extended release liquid compositions comprise cores of metformin coated with a release-controlling polymer, wherein the cores are dispersed in a suspension base. It also relates to processes for the preparation of said extended release liquid compositions.

### Background of the Invention

Metformin, an effective anti-diabetic drug known for decades, acts by reducing glucose production by the liver and by decreasing intestinal absorption of glucose. Metformin improves glucose tolerance in patients with Type II diabetes and lowers both basal and post-prandial plasma glucose.

U.S. Patent No. 8,197,850 discloses a medicament comprising microcapsules of a biguanide antihyperglycemic agent which comprises a core containing the biguanide antihyperglycemic agent and a prolonged release coating film which does not have a nitrogenous polymer, wherein the mean fraction by mass of the biguanide in the microcapsules is greater than 50%.

U.S. Patent No. 7,214,387 discloses sustained-release pharmaceutical compositions of metformin comprising xanthan gum, locust bean gum, and a diluent, wherein therapeutically beneficial blood levels of metformin are maintained over a period of time from about 1 to about 24 hours.

U.S. Patent No. 6,676,966 discloses an extended release formulation of metformin hydrochloride and an encasement coat in the form of one or more layers of pH-sensitive polymeric film, wherein said polymeric film is soluble in a pH of above 5.0.

Immediate release tablets of metformin marketed under the trade name of Glucophage® and an immediate release oral solution of metformin marketed under the trade name of Riomet® are administered multiple times a day, leading to a high level of non-compliance. Further, extended release tablets of metformin marketed under the trade name of Glucophage XR® reduces the frequency of administration, but due to the large size of the tablets, the problem of patient compliance still remains. This problem is further aggravated in patients who have difficulty swallowing, such as pediatric and geriatric patients. Furthermore, as metformin is indicated for chronic administration, such high levels of non-compliance may lead to non-adherence to the prescribed therapy, resulting in serious medical problems.

There exists a need in the art for extended release liquid compositions of metformin which provide effective control of blood glucose levels over a prolonged period of time, thereby leading to enhanced patient compliance and ease in administration. In view of this, extended release liquid compositions such as suspensions and reconstituted powder for suspensions provide the best alternative over the available dosage forms.

However, it remains a great challenge to formulate extended release liquid composition of metformin. The key hurdle remains to avoid the release of metformin into the suspension base during storage, and to begin release only when the dosage form enters the stomach. Because of its high solubility, metformin tends to leach out from the controlled release units into the suspension base during storage, thus obliterating the whole objective of the extended release. Furthermore, the irregular release may lead to subtherapeutic or toxic effects leading to serious medical conditions.

The present invention addresses this problem by providing a simplified technology which utilizes high osmotic pressure generated in the suspension base to prevent leaching of the metformin from the controlled release units into the suspension base. The present invention provides consistent *in-vitro* extended release of metformin which further ensures steady plasma concentrations throughout the shelf life of the composition.

Therefore, the present invention is a significant advance over the available dosage forms of metformin and also fulfills the long felt need to improve patient compliance by providing an extended release liquid composition of metformin.

Extended release liquid compositions of metformin of the present invention are simple, and easy to manufacture with functional reproducibility. The extended release liquid compositions are provided with a pleasant mouth feel thereby further aiding to patient compliance and ease of administration.

### Summary of the Invention

The present invention relates to extended release liquid compositions of metformin. The extended release liquid compositions are in the form of suspensions or reconstituted powder for suspensions. Said extended release liquid compositions comprise cores of metformin coated with a release-controlling polymer, wherein the cores are dispersed in a suspension base. It also relates to processes for the preparation of said extended release liquid compositions.

The extended release liquid compositions of the present invention allow for dosing flexibility based on age and body weight of the patients. Said extended release liquid compositions are stable and provide high levels of patient compliance. Also, as hypoglycemic therapy is frequently based on combinations of anti-diabetic drugs, the extended release liquid compositions of the present invention can incorporate any additional drug suitable for combination with metformin.

### Brief Description of the Drawings

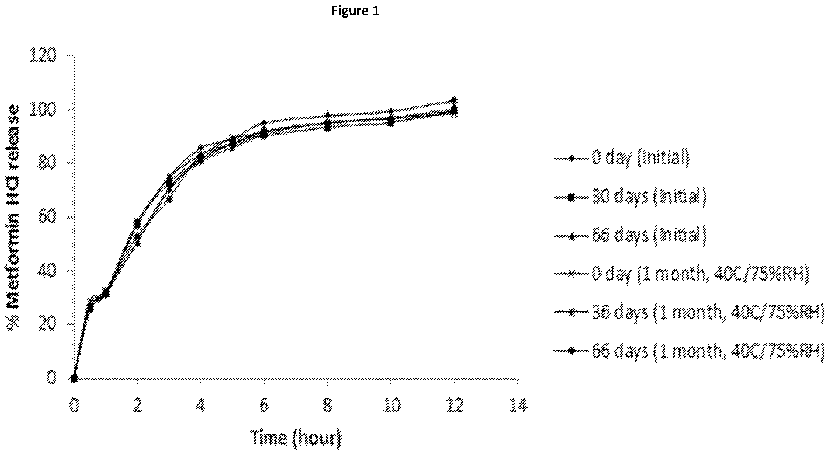

Figure 1 shows the *in-vitro* dissolution release on day 0, day 30, and day 66 of the extended release liquid composition prepared according to Example 2 upon storage at room temperature. The figure also shows the *in-vitro* dissolution release on day 0, day 36, and day 66 of the extended release liquid composition (at room temperature) formed after reconstituting the powder stored for one month at accelerated conditions.

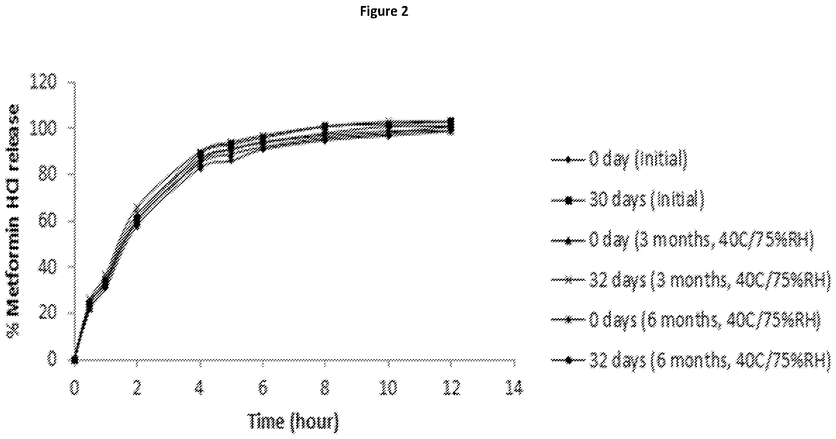

Figure 2 shows the *in-vitro* dissolution release on day 0 and day 30 of the extended release liquid composition prepared according to Example 3 upon storage at room temperature. The figure also shows the *in-vitro* dissolution release on day 0 and day 32 of the extended release liquid composition (at room temperature) formed after reconstituting the powder stored for three months and six months at accelerated conditions.

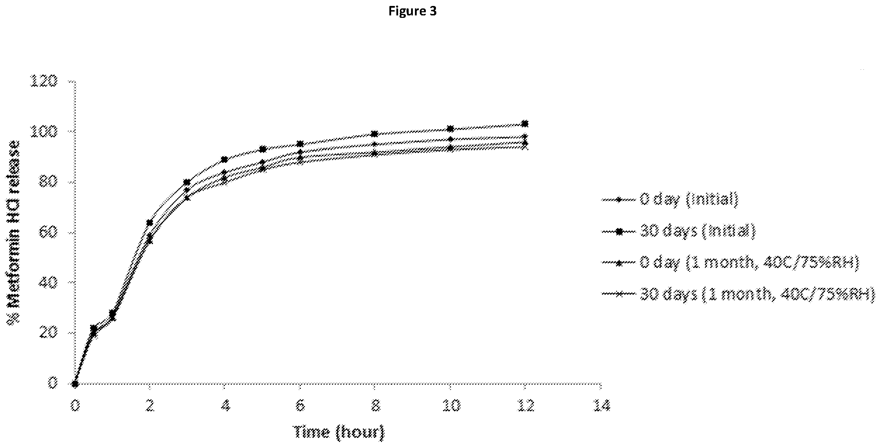

Figure 3 shows the *in-vitro* dissolution release on day 0 and day 30 of the extended release liquid composition prepared according to Example 4 upon storage at room temperature. The figure also shows the *in-vitro* dissolution release on day 0 and day 30 of the extended release liquid composition (at room temperature) formed after reconstituting the powder stored for one month at accelerated conditions.

### Detailed Description of the Invention

A first aspect of the present invention provides an extended release liquid composition of metformin comprising:
(i) cores of metformin coated with a release-controlling polymer; and
(ii) a suspension base.

According to one embodiment of the above aspect, the composition is characterized by having an *in-vitro* dissolution release profile as determined by USP type II apparatus at 100 rpm, in 1000 mL of phosphate buffer with a pH 6.8 at 37°C as follows:
- not less than 20% of metformin released after 1 hour,
- not less than 70% of metformin released after 4 hours,
- not less than 85% of metformin released after 8 hours, and
- not less than 90% of metformin released after 10 hours.

According to another embodiment of the above aspect, the *in-vitro* dissolution release profile of the extended release liquid composition remains substantially similar to the initial *in-vitro* dissolution release profile upon storage for at least seven days.

According to another embodiment of the above aspect, the extended release liquid composition is characterized by having an osmolality ratio of at least about 1. In a particular embodiment, the suspension base has an osmolality of not less than about 2 osmol/kg of the suspension base. In a more particular embodiment, the suspension base has an osmolality of not less than about 3 osmol/kg of the suspension base.

According to another embodiment of this aspect, the suspension base is responsible for creating a hypertonic environment.

According to another embodiment of the above aspect, the suspension base comprises an osmogent.

According to another embodiment of the above aspect, the extended release liquid composition is a suspension or a reconstituted powder for suspension.

According to another embodiment of the above aspect, the release-controlling polymer is selected from the group comprising a pH-dependent polymer, a pH-independent polymer, or mixtures thereof.

According to another embodiment of the above aspect, the core is in the form of a bead, a pellet, a granule, a spheroid, or the like.

According to another embodiment of the above aspect, metformin is layered onto an inert particle to form the core.

According to another embodiment of the above aspect, the extended release liquid composition has a pH ranging from about 4 to about 10.

According to another embodiment of this aspect, the extended-release liquid composition has a viscosity ranging from about 1500 to 1800 mPas/sec.

A second aspect of the present invention provides a process for the preparation of an extended release liquid composition of metformin, wherein the process comprises the steps of:
(i) preparing cores comprising metformin and one or more pharmaceutically acceptable excipients;
(ii) dissolving/dispersing a release-controlling polymer and one or more pharmaceutically acceptable coating additives in a suitable solvent;
(iii) applying the coating composition of step (ii) over the cores of step (i);
(iv) dissolving/dispersing one or more osmogents and pharmaceutically acceptable excipients in a pharmaceutically acceptable vehicle to form a suspension base; and
(v) dispersing the coated cores of step (iii) in the suspension base of step (iv) to obtain the extended release liquid composition.

A third aspect of the present invention provides a process for the preparation of an extended release liquid composition of metformin, wherein the process comprises the steps of:
(A) preparing a powder for suspension comprising the steps of:
   (i) preparing cores comprising metformin and one or more pharmaceutically acceptable excipients;
   (ii) dissolving/dispersing a release controlling polymer and one or more pharmaceutically acceptable coating additives in a suitable solvent;
   (iii) applying the coating composition of step (ii) over the cores of step (i);
   (iv) blending the coated cores of step (iii) with pharmaceutically acceptable excipients to form the powder for suspension;
(B) dissolving/dispersing one or more osmogents and pharmaceutically acceptable excipients in a pharmaceutically acceptable vehicle to form a suspension base; and
(C) reconstituting the powder for suspension of step (A) with the suspension base of step (B) to obtain the extended release liquid composition.

A fourth aspect of the present invention provides a process for the preparation an extended release liquid composition, wherein the process comprises the steps of:
(A) preparing a powder for suspension comprising the steps of:
   (i) preparing cores comprising metformin and one or more pharmaceutically acceptable excipients;
   (ii) dissolving/dispersing a release-controlling polymer and one or more pharmaceutically acceptable coating additives in a suitable solvent;
   (iii) applying the coating composition of step (ii) over the cores of step (i);
   (iv) mixing one or more osmogents and one or more pharmaceutically (i) acceptable excipients with the coated cores of step (iii) to obtain the powder for suspension; and
(B) reconstituting the powder for suspension of step (A) with a pharmaceutically acceptable vehicle to obtain the extended release liquid composition.

A fifth aspect of the present invention provides a method of treating type II diabetes by administering an extended release liquid composition of metformin comprising:
(i) cores of metformin coated with a release-controlling polymer; and
(ii) a suspension base
   wherein the composition is characterized in having an *in-vitro* dissolution release profile as determined by USP type II apparatus at 100 rpm, in 1000 mL of phosphate buffer with a pH 6.8 at 37°C as:
   - not less than 20% of metformin released after 1 hour,
   - not less than 70% of metformin released after 4 hours,
   - not less than 85% of metformin released after 8 hours, and
   - not less than 90% of metformin released after 10 hours.

According to one embodiment of this aspect, the extended release liquid composition of metformin is administered once or twice daily.

According to another embodiment of the above aspect, the extended release liquid composition further comprises one or more anti-diabetic drug selected from the group comprising acarbose, miglitol, voglibose, repaglinide, nateglinide, glibenclamide, glimepride, glipizide, gliclazide, chloropropamide, tolbutamide, phenformin, aloglitin, sitagliptin, linagliptin, saxagliptin, rosiglitazone, pioglitazone, troglitazone, faraglitazar, englitazone, darglitazone, isaglitazone, zorglitazone, liraglutide, muraglitazar, peliglitazar, tesaglitazar, canagliflozin, dapagliflozin, remogliflozin, sergliflozin, or mixtures thereof.

The term "extended release," as used herein, refers to a release profile of metformin over an extended period of time, *e.g*., over a period of 4, 6, 8, 12, 24 hours, or more.

The term "hypertonic environment," as used herein, means the suspension base has higher solute concentration which helps to generate high osmotic pressure such that there is no leaching of metformin from the controlled release coated cores into the suspension base. In the present invention, the solutes are osmogents, *i.e.,* pharmaceutically acceptable inert water-soluble compounds that contribute towards generating hypertonic environment in the suspension base.

The term "osmolality ratio," as used herein, means the ratio of osmolality of the external phase to the osmolality of the internal phase. The external phase herein means the suspension base without multiple coated cores of metformin hydrochloride. The internal phase herein means the coated cores. The osmolality of the internal phase is represented as the osmolality of the solution which prevents significant leaching of metformin from the coated cores into the solution. The leaching of metformin from the coated cores is determined by the difference in the osmolalities across the coating layer and the absence of any significant leaching from the coated cores directs that the osmolality of the solution has become equal to the osmolality of the coated cores. The osmolality ratio of the extended release suspension compositions of present invention is at least about 1.

The term "osmolality," as used herein, means the concentration of an osmogent, *i.e.,* any pharmaceutically acceptable inert water-soluble compound present in the suspension base. In the present invention, the osmolality is expressed as number of moles of any water-soluble compound per kg of a liquid phase. The liquid phase can be a suspension base or a solution. In the present invention, the osmolality may be measured according to known methods, such as using a Vapor pressure Osmometer, a Colloid Osmometer, or a Freezing Point Depression Osmometer such as OSMOMAT® 030-D or OSMOMAT® 3000, in particular by a Freezing Point Depression Osmometer.

The osmolality of suspension base of the extended release suspension compositions of the present invention remains equivalent upon storage for at least seven days. Particularly, the osmolality of the suspension base measured after one month remains equivalent to the osmolality of the suspension base measured as soon as practicable after preparation of the extended release liquid compositions. More particularly, the osmolality of the suspension base measured after three months remains equivalent to the osmolality of the suspension base measured as soon as practicable after preparation of the extended release suspension compositions. More particularly, the osmolality of the suspension base measured after three months remains substantially similar to the osmolality of the suspension base measured as soon as practicable after preparation of the extended-release liquid compositions. The equivalent osmolality of the suspension base ensures that there is no leaching of the metformin from the coated cores into the suspension base.

The *in-vitro* dissolution release profile of the extended release liquid compositions of the present invention upon storage for at least seven days remains substantially similar to the initial *in-vitro* dissolution release profile obtained as soon as practicable after preparation of the extended release liquid compositions. Particularly, the *in-vitro* dissolution release profile of the extended release liquid compositions of the present invention upon storage at room temperature for at least one month remains substantially similar to the initial *in-vitro* dissolution release profile obtained as soon as practicable after preparation of the extended release liquid compositions. More particularly, the *in-vitro* dissolution release profile of the extended release liquid compositions of the present invention upon storage at room temperature for at least three months remains substantially similar to the initial *in-vitro* dissolution release profile obtained as soon as practicable after preparation of the extended release liquid compositions. More particularly, the *in-vitro* dissolution release profile of the extended release liquid compositions of the present invention upon storage for at least six months remains substantially similar to initial *in-vitro* dissolution release profile obtained as soon as practicable after preparation of the extended release liquid compositions. The *in-vitro* dissolution release profile is measured by using any known dissolution methods, in particular the *in-vitro* dissolution release is measured at 37°C using a USP type II apparatus at 100 rpm, in 1000 mL of phosphate buffer with pH 6.8.

The extended release liquid composition of the present invention also provides the consistent *in-vivo* release which ensures steady and predictable metformin release with minimal inter and intra subject variation throughout the shelf life of the composition.

The term "substantial," as used herein refers to any value which lies within the range as defined by a variation of up to ±15 from the average value.

The term "stable," as used herein, refers to chemical stability, wherein not more than 5% w/w of total related substances are formed on storage at 40°C and 75% relative humidity (R.H.) or at 25°C and 60% R.H. for a period of at least three months to the extent necessary for the sale and use of the composition.

The extended release liquid composition of the present invention may be in the form of a suspension or a reconstituted powder for suspension. The powder for suspension may comprise of coated cores of metformin. Alternatively, powder for suspension may comprise of a mixture of coated cores of metformin, one or more osmogents, and pharmaceutically acceptable excipients. This powder for suspension may be reconstituted with a pharmaceutically acceptable vehicle or a suspension base to form an extended release liquid composition.

The term "suspension base," as used herein, refers to a medium which is used to suspend the coated cores of the metformin or to reconstitute the powder for suspension of metformin. The suspension base comprises a pharmaceutically acceptable vehicle, one or more osmogents, and pharmaceutically acceptable excipients.

The pharmaceutically acceptable vehicle as used herein means an aqueous vehicle.

The term "inert particle," as used herein, refers to a particle made from a sugar sphere also known as a non-pareil seed, a microcrystalline cellulose sphere, a dibasic calcium phosphate bead, a mannitol bead, a silica bead, a tartaric acid pellet, a wax based pellet, and the like.

The term "about," as used herein, refers to any value which lies within the range defined by a variation of up to ±10% of the value.

The term "equivalent" as used herein, refers to any value which lies within the range defined by a variation of up to ±30% of the value.

The term "significant leaching," as used herein means more than 20% of the metformin is leached out from the coated cores into the solution.

The term "metformin," as used herein, refers to metformin as well as its pharmaceutically acceptable salts, polymorphs, hydrates, solvates, prodrugs, chelates, and complexes. The preferred salt of metformin is metformin hydrochloride. The extended release liquid compositions of the present invention comprise metformin is a range of from 1% to about 30% w/w based on total weight of the composition. In particular, the extended release liquid compositions of the present invention comprise metformin is a range of from 5% to about 20% w/w based on total weight of the composition.

The extended release liquid compositions of the present invention may further include an immediate release component of metformin. The immediate release component may be present in the form a powder, pellet, a bead, spheroid, or a granule. Alternatively, the immediate release component may be present in the form of an immediate release coating over the coated cores. The immediate release component may help in providing an immediate therapeutic effect which could be subsequently followed by an extended therapeutic effect over a longer duration of time. In the present invention, the metformin may be present in the immediate release form in an amount of the about 0.5% to about 10 % w/w based on total weight of the composition, particularly in an amount of about 1% to about 5% w/w based on total weight of the composition.

The extended release liquid compositions of the present invention may further include one or more anti-diabetic drugs such as acarbose, miglitol, voglibose, repaglinide, nateglinide, glibenclamide, glimepride, glipizide, gliclazide, chloropropamide, tolbutamide, phenformin, aloglitin, sitagliptin, linagliptin, saxagliptin, rosiglitazone, pioglitazone, troglitazone, faraglitazar, englitazone, darglitazone, isaglitazone, zorglitazone, liraglutide, muraglitazar, peliglitazar, tesaglitazar, canagliflozin, dapagliflozin, remogliflozin, sergliflozin, or any other known anti-diabetic drug The extended release liquid composition of the present invention are particularly suitable for anti-diabetic drugs which are incompatible with metformin. Additionally, extended release liquid composition of the present invention can incorporate anti-diabetic drugs having a low dose e.g., glibenclamide without altering the homogeneity of the composition. These anti-diabetic drugs may be present in the form of a powder, a pellet, a bead, a spheroid, or a granule providing immediate release or in the form controlled release coated cores providing the extended release.

The extended release liquid compositions of the present invention are homogenous and delivers desired dose of metformin in every use without any risk of overdosing or underdosing.

The diameter of the cores of metformin coated with a release-controlling polymer ranges from about 10 µm to about 2000 µm, particularly from about 100 µm to about 1000 µm, and more particularly from about 150 µm to about 500 µm. Finer sizes of the coated cores help in avoiding grittiness in the mouth and thereby are easy and are more acceptable. The cores of metformin coated with a release-controlling polymer comprise metformin in an amount of about 10% to about 70% w/w based on the total weight of the coated cores, particularly from about 30% to about 50% w/w based on the total weight of the coated cores. The cores may comprise one or more pharmaceutically acceptable excipients such as binders.

The release-controlling polymers used to form the extended release coating are selected from a group comprising a pH-dependent polymer, a pH-independent polymer, or mixtures thereof.

Suitable examples of pH-dependent polymers are selected from the group comprising acrylic copolymers such as methacrylic acid and methyl methacrylate copolymers, *e.g*., Eudragit® L 100 and Eudragit® S 100. methacrylic acid and ethyl acrylate copolymers, *e.g*., Eudragit® L 100-55 and Eudragit® L 30 D-55, dimethylaminoethyl methacrylate and butyl methacrylate and methyl methacrylate copolymers *e.g*., Eudragit® E 100, Eudragit® E PO, methyl acrylate and methacrylic acid and octyl acrylate copolymers, styrene and acrylic acid copolymers, butyl acrylate and styrene and acrylic acid copolymers, and ethylacrylate-methacrylic acid copolymer; cellulose acetate phthalate; cellulose acetate succinates; hydroxyalkyl cellulose phthalates such as hydroxypropylmethyl cellulose phthalate; hydroxyalkyl cellulose acetate succinates such as hydroxypropylmethyl cellulose acetate succinate; vinyl acetate phthalates; vinyl acetate succinate; cellulose acetate trimelliate; polyvinyl derivatives such as polyvinyl acetate phthalate, polyvinyl alcohol phthalate, polyvinyl butylate phthalate, and polyvinyl acetoacetal phthalate; zein; shellac; or mixtures thereof.

Suitable examples of pH-independent polymers are selected from the group comprising cellulosic polymers such as ethyl cellulose, methyl cellulose, hydroxyethyl cellulose, hydroxypropyl cellulose, hydroxyethylmethyl cellulose, hydroxypropylmethyl cellulose, and carboxy methylcellulose; acrylic copolymers such as methacrylic acid copolymers, *e.g*., Eudragit® RS, Eudragit® RL, Eudragit® NE 30 D; cellulose acetate; polyethylene derivatives *e.g*., polyethylene glycol and polyethylene oxide; polyvinyl alcohol; polyvinyl acetate; gums *e.g*., guar gum, locust bean gum, tragacanth, carrageenan, alginic acid, gum acacia, gum arabic, gellan gum, and xanthan gum; triglycerides; waxes, *e.g*., Compritol®, Lubritab®, and Gelucires®; lipids; fatty acids or their salts/derivatives; a mixture of polyvinyl acetate and polyvinyl pyrrolidone, *e.g*., Kollidon® SR; or mixtures thereof. In particular, the pH-independent polymer used in the present invention is ethyl cellulose.

The term "osmogent," as used herein, refers to all pharmaceutically acceptable inert water-soluble compounds that can imbibe or dissolve in water and/or aqueous biological fluids. Suitable examples of osmogents or pharmaceutically acceptable inert water-soluble compounds are selected from the group comprising carbohydrates such as xylitol, mannitol, sorbitol, arabinose, ribose, xylose, glucose, fructose, mannose, galactose, sucrose, maltose, lactose, dextrose and raffinose; water-soluble salts of inorganic acids such as magnesium chloride, magnesium sulfate, potassium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, potassium dihydrogen phosphate, and sodium phosphate tribasic; water-soluble salts of organic acids such as sodium acetate, potassium acetate, magnesium succinate, sodium benzoate, sodium citrate, and sodium ascorbate; water-soluble amino acids such as glycine, leucine, alanine, methionine; urea or its derivatives; propylene glycol; glycerin; polyethylene oxide, xanthan gum, hydroxypropylmethyl cellulose; or mixtures thereof. Particularly, the osmogents used in the present invention are xylitol, mannitol, glucose, lactose, sucrose, and sodium chloride. Particularly, the osmogents used in the present invention are xylitol, mannitol, glucose, lactose, sucrose, and sodium chloride.

The term "pharmaceutically acceptable excipients," as used herein, refers to excipients that are routinely used in pharmaceutical compositions. The pharmaceutically acceptable excipients may comprise glidants, sweeteners, suspending agents, osmogents, anti-caking agents, wetting agents, preservatives, buffering agents, flavoring agents, anti-oxidants, chelating agents, or combinations thereof.

Suitable glidants are selected from the group comprising silica, calcium silicate, magnesium silicate, colloidal silicon dioxide, cornstarch, talc, stearic acid, magnesium stearate, calcium stearate, sodium stearyl fumarate, hydrogenated vegetable, or mixtures thereof.

Suitable sweeteners are selected from the group comprising saccharine or its salts such as sodium, potassium, or calcium, cyclamate or its salt, aspartame, alitame, acesulfame or its salt, stevioside, glycyrrhizin or its derivatives, sucralose, or mixtures thereof.

Suitable suspending agents are selected from the group comprising cellulose derivatives such as co-processed spray dried forms of microcrystalline cellulose and carboxymethyl cellulose sodium, hydroxypropyl cellulose, hydroxyethyl cellulose, hydroxypropylmethyl cellulose, methylcellulose, carboxymethyl cellulose and its salts/derivatives, and microcrystalline cellulose; carbomers; gums such as locust bean gum, xanthan gum, tragacanth gum, arabinogalactan gum, agar gum, gellan gum, guar gum, apricot gum, karaya gum, sterculia gum, acacia gum, gum arabic, and carrageenan; pectin; dextran; gelatin; polyethylene glycols; polyvinyl compounds such as polyvinyl acetate, polyvinyl alcohol, and polyvinyl pyrrolidone; sugar alcohols such as xylitol and mannitol; colloidal silica; or mixtures thereof. The co-processed spray dried forms of microcrystalline cellulose and carboxymethyl cellulose sodium have been marketed under the trade names Avicel® RC-501, Avicel® RC-581, Avicel® RC-591, and Avicel®CL-611.

Suitable anti-caking agents are selected from the group comprising colloidal silicon dioxide, tribasic calcium phosphate, powdered cellulose, magnesium trisilicate, starch, or mixtures thereof.

Suitable wetting agents are selected from the group comprising anionic, cationic, nonionic, or zwitterionic surfactants, or combinations thereof. Suitable examples of wetting agents are sodium lauryl sulphate; cetrimide; polyethylene glycols; polyoxyethylene-polyoxypropylene block copolymers such as poloxamers; polyglycerin fatty acid esters such as decaglyceryl monolaurate and decaglyceryl monomyristate; sorbitan fatty acid esters such as sorbitan monostearate; polyoxyethylene sorbitan fatty acid ester such as polyoxyethylene sorbitan monooleate; polyethylene glycol fatty acid ester such as polyoxyethylene monostearate; polyoxyethylene alkyl ether such as polyoxyethylene lauryl ether; polyoxyethylene castor oil; or mixtures thereof.

Suitable preservatives are selected from the group comprising parabens such as methyl paraben and propyl paraben; sodium benzoate; or mixtures thereof.

Suitable buffering agents are selected from the group comprising citric acid, sodium citrate, sodium phosphate, potassium citrate, acetate buffer, or mixtures thereof. Suitable flavoring agents are selected from the group comprising peppermint, grapefruit, orange, lime, lemon, mandarin, pineapple, strawberry, raspberry, mango, passion fruit, kiwi, apple, pear, peach, apricot, cherry, grape, banana, cranberry, blueberry, black currant, red currant, gooseberry, lingon berries, cumin, thyme, basil, camille, valerian, fennel, parsley, chamomile, tarragon, lavender, dill, bargamot, salvia, aloe vera balsam, spearmint, eucalyptus, or combinations thereof.

Suitable antioxidants are selected from the group comprising butylated hydroxytoluene (BHT), butylated hydroxyanisole (BHA), sodium metabisulfite, ascorbic acid, propyl gallate, thiourea, tocopherols, beta-carotene, or mixtures thereof.

Suitable chelating agents are selected from the group comprising ethylenediamine tetraacetic acid or derivatives/salts thereof, *e.g*., disodium edetate; dihydroxyethyl glycine; glucamine; acids, *e.g*., citric acid, tartaric acid, gluconic acid, and phosphoric acid; or mixtures thereof.

Suitable binders are selected from the group comprising polyvinyl pyrrolidone, starch, pregelatinized starch, hydroxypropylmethyl cellulose, hydroxyethyl cellulose, methyl cellulose, sodium carboxymethyl cellulose, gums, acrylate polymers, or mixtures thereof.

The cores of the present invention comprising metformin can be prepared by any method known in the art, *e.g*., extrusion-spheronoization, wet granulation, dry granulation, hot-melt extrusion granulation, spray drying, and spray congealing. Alternatively, metformin can be layered onto an inert particle to form the core.

Further, metformin can be directly coated with a release-controlling polymer to form the microparticles or microcapsules. The microparticles or microcapsules can be prepared by a process of homogenization, solvent evaporation, coacervation phase separation, spray drying, spray congealing, polymer precipitation, or supercritical fluid extraction.

The extended release liquid compositions of the present invention may further comprise one or more seal coating layers which may be applied before and/or after the functional coating layer. The seal coating layer may comprise of one or more film forming polymers and coating additives.

Examples of film-forming polymers include ethylcellulose, hydroxypropyl methylcellulose, hydroxypropylcellulose, methylcellulose, carboxymethyl cellulose, hydroxymethylcellulose, hydroxyethylcellulose, cellulose acetate, hydroxypropyl methylcellulose phthalate, cellulose acetate phthalate, cellulose acetate trimellitate; waxes such as polyethylene glycol; methacrylic acid polymers such as Eudragit®. Alternatively, commercially available coating compositions comprising film-forming polymers marketed under various trade names, such as Opadry® may also be used.

The coating additives used in the present invention are selected from the group comprising plasticizers, opacifiers, anti-tacking agents, coloring agents, or combinations thereof.

Suitable plasticizers are selected from the group comprising triethyl citrate, dibutylsebacate, triacetin, acetylated triacetin, tributyl citrate, glyceryl tributyrate, diacetylated monoglyceride, rapeseed oil, olive oil, sesame oil, acetyl tributyl citrate, acetyl triethyl citrate, glycerin, sorbitol, diethyl oxalate, diethyl phthalate, diethyl malate, diethyl fumarate, dibutyl succinate, diethyl malonate, dioctyl phthalate, or combinations thereof.

Suitable opacifiers are selected from the group comprising titanium dioxide, manganese dioxide, iron oxide, silicon dioxide, or combinations thereof.

Suitable anti-tacking agents are selected from the group comprising talc, magnesium stearate, calcium stearate, stearic acid, silica, glyceryl monostearate, or combinations thereof.

Suitable coloring agents are selected from the group consisting of FD&C (Federal Food, Drug and Cosmetic Act) approved coloring agents; natural coloring agents; natural juice concentrates; pigments such as iron oxide, titanium dioxide, and zinc oxide; or combinations thereof.

Coating may be performed by applying the coating composition as a solution/suspension/blend using any conventional coating technique known in the art, such as spray coating in a conventional coating pan, fluidized bed processor, dip coating, or compression coating. The percentage of the coating build-up shall be varied depending on the required extended release.

Suitable solvents used for granulation or for forming a solution or dispersion for coating are selected from the group comprising water, ethanol, methylene chloride, isopropyl alcohol, acetone, methanol, or combinations thereof.

The extended release liquid compositions of the present invention may be packaged in a suitable package such as a bottle. The powder for suspension may be packaged in a suitable package such as a bottle or a sachet. Further, the sachet can be filled as a unit dose or a multidose sachet. The present invention further includes a co-package or a kit comprising two components, wherein one package or one component comprises a powder for suspension and another package or another component comprises a suspension base or a pharmaceutically acceptable vehicle.

The invention may be further illustrated by the following example, which is for illustrative purposes only and should not be construed as limiting the scope of the invention in any way.

### EXAMPLES

### Example 1

| **Ingredients** | **Quantity (mg/mL)** |
|---|---|
| **Core** | |
| Metformin hydrochloride | 80.00 |
| Microcrystalline cellulose spheres | 56.00 |
| Hydroxypropylmethyl cellulose | 4.00 |
| Purified water | q.s. |

| **Extended Release Coating** | |
|---|---|
| Ethyl cellulose | 45.00 |
| Dibutyl sebacate | 1.50 |
| Acetone | q.s. |
| Purified water | q.s. |

| **Total Weight of Extended Release Beads** | **186.50 mg** |
|---|---|
| Metformin hydrochloride | 20.00 |
| Xylitol | 450.00 |
| Microcrystalline cellulose - sodium carboxymethyl cellulose (Avicel® CL-611 ) | 20.00 |
| Xanthan gum | 1.50 |
| Strawberry flavor | 2.00 |
| Sucralose | 0.50 |
| Sodium benzoate | 3.00 |
| Colloidal silicon dioxide | 3.50 |

| Vehicle | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Metformin hydrochloride, xylitol, microcrystalline cellulose - sodium carboxymethyl cellulose, xanthan gum, strawberry flavor, sucralose, sodium benzoate, and colloidal silicon dioxide were mixed.
6. The coated beads of step 4 were mixed with the mixture of step 5 to obtain a powder for suspension.
7. The powder for suspension of step 6 is reconstituted with purified water when required to form the extended release liquid composition.

### In-Vitro Studies

The extended release liquid composition prepared as per Example 1 was stored at room temperature for 66 days. The *in-vitro* dissolution was determined at 0, 30, and 66 days using USP type II apparatus at 100 rpm, in 1000 mL of phosphate buffer with pH 6.8 at 37°C. The results of the release studies are represented in Table 1.

**Table 1: Percentage (%) of the In-Vitro Metformin Release in USP Type II Apparatus (Media: Phosphate Buffer, pH 6.8, 1000 mL, and 100 rpm)**

| **Number of Days** | **0** | **30** | **66** |
|---|---|---|---|
| **Time (hours)** | **Percentage of Metformin Release** | | |
| 0.5 | 27.0 | 25.6 | 26.3 |
| 1 | 30.9 | 31.5 | 31.6 |
| 2 | 56.9 | 58.3 | 50.9 |
| 3 | 74.9 | 72.8 | 70.6 |
| 4 | 85.6 | 81.7 | 81.6 |
| 5 | 89.1 | 87.7 | 87.4 |
| 6 | 94.9 | 90.3 | 92.3 |
| 8 | 97.7 | 93.5 | - |
| 10 | 99.4 | 95.3 | - |
| 12 | 103.4 | 99.4 | 100.0 |

From the above data, it is clear that the extended release liquid composition prepared according to Example 1 provides substantially similar *in-vitro* metformin release for 66 days.

The powder for suspension prepared as per Example 1 (tuntil step 6) was kept for one month at accelerated conditions *i.e.,* 40°C/75% R.H. After one month, the powder for suspension was reconstituted with required amount of purified water and this extended release suspensions composition was kept for 66 days at room temperature. The *in-vitro* dissolution was determined at 0, 30, and 66 days using USP type II apparatus at 100 rpm, in 1000 mL of phosphate buffer with pH 6.8 at 37°C. The results of the release studies are represented in Table 2.

**Table 2: Percentage (%) of the In-Vitro Metformin Release in USP Type II Apparatus (Media: Phosphate Buffer, pH 6.8, 1000 mL, and 100 rpm)**

| **Number of Days After Reconstitution** | **0** | **36** | **66** |
|---|---|---|---|
| **Time (hours)** | **Percentage of Metformin Release** | | |
| 0.5 | 28.8 | 26.2 | 27.0 |
| 1 | 32.4 | 33.0 | 32.0 |
| 2 | 57.6 | 50.5 | 53.0 |
| 3 | 74.8 | 70.3 | 67.0 |
| 4 | 83.1 | 80.7 | 83.0 |
| 5 | 89.2 | 85.9 | 87.0 |
| 6 | 91.3 | 91.2 | 92.0 |
| 8 | 95.2 | - | 95.0 |
| 10 | 96.6 | - | 97.0 |
| 12 | 98.6 | 101.3 | 100.0 |

From the above data, it is clear that the extended release powder prepared according to Example 1 stored at accelerated conditions for one month, upon reconstitution and storage for 66 days at room temperature provides substantially similar *in-vitro* metformin release for 66 days. The results are shown in Figure 1.

### Example 2

| **Ingredients** | **Quantity (mg/mL)** |
|---|---|
| **Core** | |
| Metformin hydrochloride | 80.00 |
| Microcrystalline cellulose spheres | 56.00 |
| Hydroxypropylmethyl cellulose | 4.00 |
| Purified water | q.s. |

| **Extended Release Coating** | |
|---|---|
| Ethyl cellulose | 50.40 |
| Dibutyl sebacate | 5.60 |
| Acetone | q.s. |
| Purified water | q.s. |

| **Total Weight of Extended Release Beads** | **196.00 mg** |
|---|---|
| Metformin hydrochloride | 20.00 |
| Xylitol | 450.00 |
| Microcrystalline cellulose - sodium carboxymethyl cellulose (Avicel® CL-611) | 20.00 |
| Xanthan gum | 1.50 |
| Strawberry flavor | 2.00 |
| Sucralose | 0.50 |

| Vehicle | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Metformin hydrochloride, xylitol, microcrystalline cellulose - sodium carboxymethyl cellulose, xanthan gum, strawberry flavor, and sucralose were mixed.
6. The coated beads of step 4 were mixed with the mixture of step 5 to form a powder for suspension.
7. The powder for suspension of step 6 is reconstituted with purified water when required to form the extended release liquid composition.

### In-Vitro Studies

The extended release liquid composition prepared as per Example 2 was stored at room temperature for 30 days. The *in-vitro* dissolution was determined at 0 and 30 days using a USP type II apparatus at 100 rpm, in 1000 mL of phosphate buffer with pH 6.8 at 37°C. The results of the release studies are represented in Table 3.

**Table 3: Percentage (%) of the In-Vitro Metformin Release in USP Type II Apparatus (Media: Phosphate Buffer, pH 6.8, 1000 mL, and 100 rpm)**

| **Number of Days** | **0** | 30 |
|---|---|---|
| **Time (hours)** | **Percentage** of Metformin Release | |
| 0.5 | 22 | 24 |
| 1 | 31 | 34 |
| 2 | 58 | 61 |
| 4 | 83 | 89 |
| 5 | 86 | 93 |
| 6 | 91 | 96 |
| 8 | 95 | 101 |
| 10 | 97 | 102 |
| 12 | 99 | 103 |

From the above data, it is clear that the extended release liquid composition prepared according to Example 2 provides a substantially similar *in-vitro* metformin release profile for 30 days.

The powder for suspension prepared as per Example 2 (until step 6) was kept for three months at accelerated conditions 40°C/75% R.H. After three months, the powder for suspension was reconstituted with required amount of purified water and this extended release suspensions composition was kept for 30 days at room temperature. The *in-vitro* dissolution was determined at 0 and 32 days using USP type II apparatus at 100 rpm, in 1000 mL of phosphate buffer with pH 6.8 at 37°C. The results of the release studies are represented in Table 4.

**Table 4: Percentage (%) of the In-Vitro Metformin Release in USP Type II Apparatus (Media: Phosphate Buffer, pH 6.8, 1000 mL, and 100 rpm)**

| **Number of Days After Reconstitution** | **0** | **32** |
|---|---|---|
| **Time (hours)** | **Percentage of Metformin Release** | |
| 0.5 | 22 | 26 |
| 1 | 33 | 37 |
| 2 | 60 | 66 |
| 4 | 85 | 90 |
| 5 | 89 | 94 |
| 6 | 92 | 97 |
| 8 | 96 | 101 |
| 10 | 98 | 103 |
| 12 | 101 | 103 |

The powder for suspension prepared as per Example 2 (till step 6) was kept for six months at accelerated conditions *i.e.,* 40°C/75% R.H. After six months, the powder for suspension was reconstituted with required amount of purified water and this extended release suspensions composition was kept for 32 days at room temperature. The *in-vitro* dissolution was determined at 0 and 32 days using USP type II apparatus at 100 rpm, in 1000 mL of phosphate buffer with pH 6.8 at 37°C. The results of the release studies are represented in Table 5.

**Table 5: Percentage (%) of the In-Vitro Metformin Release in USP Type II Apparatus (Media: Phosphate Buffer, pH 6.8, 1000 mL, and 100 rpm)**

| **Number of Days After Reconstitution** | **0** | **32** |
|---|---|---|
| **Time (hours)** | **Percentage of Metformin Release** | |
| 0.5 | 24 | 25 |
| 1 | 35 | 34 |
| 2 | 63 | 60 |
| 4 | 87 | 86 |
| 5 | 91 | 91 |
| 6 | 94 | 94 |
| 8 | 97 | 98 |
| 10 | 99 | 101 |
| 12 | 99 | 101 |

From the above data, it is clear that the extended release powder prepared according to Example 2 and stored at accelerated conditions for three or six months, upon reconstitution and storage for 30 days at room temperature, provides a substantially similar *in-vitro* metformin release for 30 days. The results are presented in Figure 2.

### Example 3

| **Ingredients** | **Quantity (mg/mL)** |
|---|---|
| **Core** | |
| Metformin hydrochloride | 80.00 |
| Microcrystalline cellulose spheres | 56.00 |
| Hydroxypropylmethyl cellulose | 4.00 |
| Purified water | q.s. |

| **Extended Release Coating** | |
|---|---|
| Ethyl cellulose | 61.48 |
| Dibutyl sebacate | 1.52 |
| Acetone | q.s. |
| Purified water | q.s. |

| **Total Weight of Extended Release Beads** | 203.00 mg |
|---|---|
| Metformin hydrochloride | 20.00 |
| Xylitol | 450.00 |
| Microcrystalline cellulose - sodium carboxymethyl cellulose (Avicel® CL-611) | 20.00 |
| Xanthan gum | 1.50 |
| Strawberry flavor | 2.00 |
| Sucralose | 0.50 |
| Colloidal silicon dioxide | 3.50 |

| Vehicle | |
|---|---|
| Purified water | q.s. to 1 mL |

### Procedure:

1. Metformin hydrochloride and hydroxypropylmethyl cellulose were dissolved in purified water.
2. Microcrystalline cellulose spheres were coated with the solution of step 1.
3. Ethyl cellulose and dibutyl sebacate were dispersed in a mixture of acetone and purified water.
4. The beads of step 2 were coated with the coating dispersion of step 3.
5. Metformin hydrochloride, xylitol, microcrystalline cellulose - sodium carboxymethyl cellulose, xanthan gum, strawberry flavor, sucralose, and colloidal silicon dioxide were mixed.
6. The coated beads of step 4 were mixed with the mixture of step 5 to form a powder for suspension.
7. The powder for suspension of step 6 is reconstituted with required amount of purified water when required to form the extended release liquid composition.

### In-Vitro Studies

The extended release liquid composition prepared as per Example 3 was stored at room temperature for 30 days. The *in-vitro* dissolution was determined at 0 and 30 days using a USP type II apparatus at 100 rpm, in 1000 mL of phosphate buffer with pH 6.8 at 37°C. The results of the release studies are represented in Table 6.

**Table 6: Percentage (%) of the In-Vitro Metformin Release in USP Type II Apparatus (Media: Phosphate Buffer, pH 6.8, 1000 mL, and 100 rpm)**

| **Number of Days** | **0** | **30** |
|---|---|---|
| **Time (hours)** | **Percentage of Metformin Release** | |
| 0.5 | 20 | 22 |
| 1 | 27 | 28 |
| 2 | 59 | 64 |
| 3 | 77 | 80 |
| 4 | 84 | 89 |
| 5 | 88 | 93 |
| 6 | 92 | 95 |
| 8 | 95 | 99 |
| 10 | 97 | 101 |
| 12 | 98 | 103 |

From the above *in-vitro* release data, it is evident that the extended release liquid composition prepared according to Example 3 provides the substantially similar *in-vitro* release for 30 days.

The powder for suspension prepared as per Example 3 (till step 6) was kept for one month at accelerated conditions *i.e.,* 40°C/75% R.H. After one month, the powder for suspension was reconstituted with purified water and kept for 30 days at room temperature. The *in-vitro* dissolution profile was determined at 0 and 30 days using a USP type II apparatus at 100 rpm, in 1000 mL of phosphate buffer with pH 6.8 at 37°C. The results of the release studies are represented in Table 7.

**Table 7: Percentage (%) of the In-Vitro Metformin Release in USP Type II Apparatus (Media: Phosphate Buffer, pH 6.8, 1000 mL, and 100 rpm)**

| **Number of Days After Reconstitution** | **0** | **30** |
|---|---|---|
| **Time (hours)** | **Percentage of Metformin Release** | |
| 0.5 | 20 | 19 |
| 1 | 26 | 26 |
| 2 | 57 | 57 |
| 3 | 74 | 74 |
| 4 | 82 | 80 |
| 5 | 86 | 85 |
| 6 | 90 | 88 |
| 8 | 92 | 91 |
| 10 | 94 | 93 |
| 12 | 96 | 94 |

From the above data, it is clear that the extended release powder prepared according to Example 3 stored at accelerated condition for one month, upon reconstitution and storage for 30 days at room temperature provides substantially similar *in-vitro* metformin release for 30 days. The results are presented in Figure 3.

### Osmolality Measurement of the Extended Release Suspension

The metformin extended release powder prepared according to the Example 3 (till step 6) was reconstituted with purified water. This suspension was shaken manually for at least 20 minutes. This suspension was then filtered and diluted with purified water and the osmolality was measured using OSMOMAT® 030-D.

The osmolality of the suspension base was found to be 4.112 osmol/kg of the suspension base on day 0.

The osmolality of the suspension base was found to be 4.328 osmol/kg of the suspension base on day 7.

It is evident from the above data that the osmolality of the suspension base of the extended release suspension composition as per Example 3 remains equivalent for seven days.

### Osmolality Measurement of the External Phase

The metformin hydrochloride, xylitol, microcrystalline cellulose - sodium carboxymethyl cellulose, xanthan gum, strawberry flavor, sucralose, and colloidal silicon dioxide were mixed as per step 6 of Example 3. This powder was reconstituted with purified water. This suspension was then filtered and diluted with purified water and the osmolality was measured using OSMOMAT® 030-D.

The osmolality of the suspension base *i.e.,* external phase was found to be 4.204 osmol/kg of the suspension base.

### Osmolality Measurement of the Internal Phase

Various solutions having various concentrations of osmogent (sodium chloride) were prepared as per Examples 3A-3F. The osmolalities of these solutions were measured using OSMOMAT® 030-D.

| **Ingredient** | **Example 3A** | **Example 3B** | **Example 3C** | **Example 3D** | **Example 3E** | **Example 3F** |
|---|---|---|---|---|---|---|
| Sodium Chloride (mg) | 30.00 | 60.00 | 120.00 | 180.00 | 240.00 | 300.00 |
| Purified water | q.s. to 1 mL | q.s. to 1 mL | q.s. to 1 mL | q.s. to 7.5 mL | q.s. to 1 mL | q.s. to 1 mL |
| Osmolality (osmol/kg) | 0.910 | 1.787 | 3.574* | 5.361* | 7.148* | 8.935* |

| | | | | | | |
|---|---|---|---|---|---|---|
| * Extrapolated using values of dilute solutions | | | | | | |

The coated beads of step 4 of Example 3 were dispersed in different solutions as per Examples 3A-3F. These solutions were kept for seven days at room temperature. After seven days, each solution was analyzed by HPLC for metformin content. The results are represented in Table 8.

**Table 8: Effect of Osmolality on Metformin Leaching**

| **Example** | **Osmolality (osmol/kg) of the solution** | **Metformin Content (%)** |
|---|---|---|
| 3A | 0.910 | 67.3 |
| 3B | 1.787 | 30.3 |
| 3C | 3.574* | 2.9 |
| 3D | 5.361* | 1.8 |
| 3E | 7.148* | 1.7 |
| 3F | 8.935* | 1.0 |

| | | |
|---|---|---|
| * Extrapolated using values of dilute solutions | | |

From the above data, it is evident that the leaching of metformin from the coated beads into the solution was decreasing as the osmolality of the solution was increasing from Examples 3A-3F. The leaching is found to be significantly reduced from Example 3C onwards. The osmolality of the formulation prepared according to Example 3C is considered to be the osmolality of the internal phase.

### Osmolality Ratio 1.176

### Dose Uniformity Data

The extended release suspension equivalent to 100 mL was prepared according to formula given in Example 3. This suspension was shaken manually for at least 20 minutes and then ten 7.5 mL samples were taken with a graduated syringe. The metformin content of each sample is determined by HPLC method [Inertsil ODS column (250 x 4.6 mm, 5 µm); mobile phase-buffer (pH 3.5):acetonitrile (95:5 v/v); flow rate of 1.5 mL/min; UV detection at 233 nm] The results are shown in Table 9.

**Table 9: Metformin Content (%) For Each 7.5 mL of Suspension**

| **Sample Number** | **Metformin content for 7.5 mL of suspension (%)** |
|---|---|
| 1 | 98.6 |
| 2 | 97.9 |
| 3 | 96.6 |
| 4 | 97.2 |
| 5 | 99.7 |
| 6 | 96.4 |
| 7 | 95.9 |
| 8 | 97.3 |
| 9 | 98.8 |
| 10 | 96.9 |
| Mean value | 97.5 |

Form the above data, it is evident that the extended release liquid composition prepared according to Example 3 is homogeneous.

### Assay Data

The assay for the extended release liquid composition prepared as per Example 3 was determined at 0 day and after storage at room temperature for 30 days. The powder for suspension prepared as per Example 3 (till step 7) was kept for one month at accelerated conditions *i.e.,* 40°C/75% R.H. After one month, the powder for suspension was reconstituted with purified water and then the assay was determined at day 0 and after storage at room temperature for 30 days. The assay of metformin was determined by HPLC [Inertsil ODS column (250 x 4.6 mm, 5 µm); mobile phase-buffer (pH 3.5):acetonitrile (95:5 v/v); flow rate of 1.5 mL/min; UV detection at 233 nm]. The results are shown in Table 10.

**Table 10: Assay for Metformin**

| Condition | % **Assay (After reconstitution)** | |
|---|---|---|
| | 0 day | 30 days |
| Initial | 97.0 | 99.5 |
| 1 month (40°C/75% R.H.) | 97.4 | 98.9 |

It is evident from the above data that the extended release liquid composition prepared as per Example 3 is stable.

The *in-vitro* dissolution of the formulation prepared according to Example 3 (7.5 mL of extended release suspension equivalent to 750 mg of metformin hydrochloride) was compared with commercially available Glucophage® XR Tablets 750 mg. The *in-vitro* dissolution was determined by using a USP type II apparatus at 50 rpm, in 900 mL of phosphate buffer with pH 6.8 at 37°C. The results of the release studies are represented in Table 11.

**Table 11: Comparative Dissolution Release Data of Example 3 and Glucophage® XR Tablets**

| **Time (hours)** | **Percentage of Metformin Release from Example 3** | **Percentage of Metformin Release from Glucophage® XR Tablets** |
|---|---|---|
| 0.5 | 18 | 21 |
| 1 | 22 | 33 |
| 2 | 45 | 49 |
| 3 | 66 | 61 |
| 4 | 76 | 70 |
| 5 | 83 | 77 |
| 6 | 86 | 83 |
| 8 | 90 | 90 |
| 10 | 92 | 94 |
| 12 | 94 | 96 |

## Claims

1. An extended release liquid composition of metformin comprising;
(i) cores of metformin coated with a release controlling polymer; and
(ii) a suspension base;
wherein the composition is a suspension or a reconstituted powder for suspension **characterized by** having the *in-vitro* dissolution release profile as determined by USP type II apparatus at 100 rpm, in 1000 mL of phosphate buffer with a pH 6.8 at 37°C as follows:
- not less than 20% of metformin released after 1 hour,
- not less than 70% of metformin released after 4 hours,
- not less than 85% of metformin released after 8 hours, and
- not less than 90% of metformin released after 10 hours,
and wherein an *in-vitro* dissolution release profile of the extended release liquid composition upon storage for at least seven days remains substantially similar to the initial *in-vitro* dissolution release profile.

2. The extended release liquid composition of claim 1, wherein the composition is **characterized by** having an osmolality ratio of at least about 1.

3. The extended release liquid composition of claim 1, wherein the suspension base comprises an osmogent.

4. The extended release liquid composition of claim 1, wherein the composition is a suspension in particular a suspension reconstituted from a powder for suspension whose *in vitro* dissolution rate is measured upon constitution of the suspension.

5. The extended release liquid composition of claim 1, wherein the composition has a pH ranging from about 4 to about 10.

6. The extended release liquid composition of claim 1, wherein the metformin is layered onto an inert particle to form the core.

7. The extended release liquid composition of claim 6, wherein the inert particle is selected from a group comprising a non-pareil seed, a microcrystalline cellulose sphere, a dibasic calcium phosphate bead, a mannitol bead, a silica bead, a tartaric acid pellet, or a wax based pellet.

8. The extended release liquid composition of claim 3, wherein the osmogent is selected from the group comprising carbohydrates such as xylitol, mannitol, sorbitol, arabinose, ribose, xylose, glucose, fructose, mannose, galactose, sucrose, maltose, lactose, dextrose and raffinose; water-soluble salts of inorganic acids such as magnesium chloride, magnesium sulfate, potassium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, potassium dihydrogen phosphate, and sodium phosphate tribasic; water-soluble salts of organic acids such as sodium acetate, potassium acetate, magnesium succinate, sodium benzoate, sodium citrate, and sodium ascorbate; water-soluble amino acids such as glycine, leucine, alanine, methionine; urea or its derivatives; propylene glycol; glycerin; or mixtures thereof.

9. The extended release liquid composition of claim 1, wherein the release-controlling polymer is selected from the group comprising a pH-dependent polymer, a pH-independent polymer, or mixtures thereof.

10. The extended release liquid composition of claim 9, wherein the pH-dependent polymer is selected from the group comprising acrylic copolymers such as methacrylic acid and methyl methacrylate copolymers, *e.g*., Eudragit® L 100 and Eudragit® S 100, methacrylic acid and ethyl acrylate copolymers, *e.g*., Eudragit® L 100-55 and Eudragit® L 30 D-55, dimethylaminoethyl methacrylate and butyl methacrylate and methyl methacrylate copolymer *e.g*., Eudragit® E 100, Eudragit® E PO, methyl acrylate and methacrylic acid and octyl acrylate copolymers, styrene and acrylic acid copolymers, butyl acrylate and styrene and acrylic acid copolymers, and ethylacrylate-methacrylic acid copolymer; cellulose acetate phthalate; cellulose acetate succinates; hydroxyalkyl cellulose phthalates such as hydroxypropylmethyl cellulose phthalate; hydroxyalkyl cellulose acetate succinates such as hydroxypropylmethyl cellulose acetate succinate; vinyl acetate phthalates; vinyl acetate succinate; cellulose acetate trimelliate; polyvinyl derivatives such as polyvinyl acetate phthalate, polyvinyl alcohol phthalate, polyvinyl butylate phthalate, and polyvinyl acetoacetal phthalate; zein; shellac; or mixtures thereof.

11. The extended release liquid composition of claim 9, wherein the pH-independent polymer is selected from the group comprising cellulosic polymers such as ethyl cellulose, methyl cellulose, hydroxyethyl cellulose, hydroxypropyl cellulose, hydroxyethylmethyl cellulose, hydroxypropylmethyl cellulose, and carboxy methylcellulose; acrylic copolymers such as methacrylic acid copolymers, *e.g*., Eudragit® RS, Eudragit® RL, Eudragit®NE 30 D; cellulose acetate; polyethylene derivatives *e.g*., polyethylene glycol and polyethylene oxide; polyvinyl alcohol; polyvinyl acetate; gums *e.g*., guar gum, locust bean gum, tragacanth, carrageenan, alginic acid, gum acacia, gum arabic, gellan gum, and xanthan gum; triglycerides; waxes, *e.g*., Compritol®, Lubritab®, and Gelucires®; lipids; fatty acids or their salts/derivatives; a mixture of polyvinyl acetate and polyvinyl pyrrolidone, *e.g*., Kollidon® SR; or mixtures thereof.

12. The extended release liquid composition of claim 1, wherein the suspension base further comprises one or more pharmaceutically acceptable excipients selected from the group comprising suspending agents, anti-caking agents, wetting agents, preservatives, buffering agents, flavoring agents, anti-oxidants, and chelating agents.

13. The extended release liquid composition of claim 1, wherein the composition further includes metformin in an immediate release form.

14. The extended release liquid composition of claim 1, wherein the composition further includes an additional anti-diabetic drug.

15. The extended release liquid composition of claim 1, for use in treating type II diabetes by administration once or twice daily.

16. The extended release liquid composition of claim 15, which further comprises one or more anti-diabetic drugs selected from the group comprising acarbose, miglitol, voglibose, repaglinide, nateglinide, glibenclamide, glimepride, glipizide, gliclazide, chloropropamide, tolbutamide, phenformin, aloglitin, sitagliptin, linagliptin, saxagliptin, rosiglitazone, pioglitazone, troglitazone, faraglitazar, englitazone, darglitazone, isaglitazone, zorglitazone, liraglutide, muraglitazar, peliglitazar, tesaglitazar, canagliflozin, dapagliflozin, remogliflozin, sergliflozin, or mixtures thereof.

## Patentansprüche

1. Flüssige Zusammensetzung von Metformin mit verlängerter Freisetzung, umfassend:
(i) Kerne aus Metformin, überzogen mit einem freisetzungssteuernden Polymer; und
(ii) eine Suspensionsgrundlage;
wobei die Zusammensetzung eine Suspension oder ein rekonstituiertes Pulver zur Suspension ist, **dadurch gekennzeichnet, dass** sie das In-vitro-Auflösungs-Freisetzungsprofil, wie bestimmt mittels USP-Apparatur vom Typ II bei 100 U/min in 1000 ml Phosphatpuffer mit einem pH-Wert von 6,8 bei 37 °C, wie folgt aufweist:
- nicht weniger als 20 % Metformin nach 1 Stunde freigesetzt,
- nicht weniger als 70 % Metformin nach 4 Stunden freigesetzt,
- nicht weniger als 85 % Metformin nach 8 Stunden freigesetzt und
- nicht weniger als 90 % Metformin nach 10 Stunden freigesetzt,
und wobei ein *In-vitro-*Auflösungs-Freisetzungsprofil der flüssigen Zusammensetzung mit verlängerter Freisetzung nach Lagerung während zumindest sieben Tagen dem anfänglichen *In-vitro*-Auflösungs-Freisetzungsprofil im Wesentlichen ähnlich bleibt.

2. Flüssige Zusammensetzung mit verlängerter Freisetzung nach Anspruch 1, wobei die Zusammensetzung **dadurch gekennzeichnet ist, dass** sie ein Osmolalitätsverhältnis von zumindest etwa 1 aufweist.

3. Flüssige Zusammensetzung mit verlängerter Freisetzung nach Anspruch 1, wobei die Suspensionsgrundlage ein osmotisches Mittel umfasst.

4. Flüssige Zusammensetzung mit verlängerter Freisetzung nach Anspruch 1, wobei die Zusammensetzung eine Suspension, insbesondere eine Suspension ist, die aus einem Pulver zur Suspension rekonstituiert ist, dessen *In-vitro-*Auflösungsgeschwindigkeit bei Konstitution der Suspension gemessen ist.

5. Flüssige Zusammensetzung mit verlängerter Freisetzung nach Anspruch 1, wobei die Zusammensetzung einen pH-Wert im Bereich von etwa 4 bis etwa 10 aufweist.

6. Flüssige Zusammensetzung mit verlängerter Freisetzung nach Anspruch 1, wobei das Metformin auf ein inertes Teilchen geschichtet ist, um den Kern zu bilden.

7. Flüssige Zusammensetzung mit verlängerter Freisetzung nach Anspruch 6, wobei das inerte Teilchen aus einer Gruppe ausgewählt ist, die eine Nonpareille, eine mikrokristalline Cellulosekugel, ein Kügelchen aus zweibasigem Calciumphosphat, ein Mannitkügelchen, ein Siliciumdioxidkügelchen, ein Weinsäurepellet oder ein Pellet auf Wachsgrundlage umfasst.

8. Flüssige Zusammensetzung mit verlängerter Freisetzung nach Anspruch 3, wobei das osmotische Mittel aus der Gruppe ausgewählt ist, welche umfasst: Kohlenhydrate wie Xylit, Mannit, Sorbit, Arabinose, Ribose, Xylose, Glucose, Fructose, Mannose, Galactose, Sucrose, Maltose, Lactose, Dextrose und Raffinose; wasserlösliche Salze von anorganischen Säuren wie Magnesiumchlorid, Magnesiumsulfat, Kaliumsulfat, Lithiumchlorid, Natriumchlorid, Kaliumchlorid, Lithiumhydrogenphosphat, Natriumhydrogenphosphat, Kaliumhydrogenphosphat, Lithiumdihydrogenphosphat, Natriumdihydrogenphosphat, Kaliumdihydrogenphosphat und Natriumphosphat dreibasig; wasserlösliche Salze von organischen Säuren wie Natriumacetat, Kaliumacetat, Magnesiumsuccinat, Natriumbenzoat, Natriumcitrat und Natriumascorbat; wasserlösliche Aminosäuren wie Glycin, Leucin, Alanin, Methionin, Harnstoff oder dessen Derivate; Propylenglycol; Glycerin; oder Gemische davon.

9. Flüssige Zusammensetzung mit verlängerter Freisetzung nach Anspruch 1, wobei das freisetzungssteuernde Polymer aus der Gruppe ausgewählt ist, die ein pH-Wert-abhängiges Polymer, ein pH-Wert-unabhängiges Polymer oder Gemische davon umfasst.

10. Flüssige Zusammensetzung mit verlängerter Freisetzung nach Anspruch 9, wobei das pH-Wert-abhängige Polymer aus der Gruppe ausgewählt ist, welche umfasst: Acrylcopolymere wie Copolymere aus Methacrylsäure und Methylmethacrylat, *z*. *B.* Eudragit® L 100 und Eudragit® S 100, Copolymere aus Methacrylsäure und Ethylacrylat, *z*. *B.* Eudragit® L 100-55 und Eudragit® L 30 D-55, Copolymer aus Dimethylaminoethylmethacrylat und Butylmethacrylat und Methylmethacrylat, *z*. *B.* Eudragit® E 100, Eudragit® E PO, Copolymere aus Methylacrylat und Methacrylsäure und Octylacrylat, Copolymere aus Styrol und Acrylsäure, Copolymere aus Butylacrylat und Styrol und Acrylsäure, und Ethylacrylat-Methacrylsäure-Copolymer; Celluloseacetatphthalat; Celluloseacetatsuccinate; Hydroxyalkylcellulosephthalate wie Hydroxypropylmethylcellulosephthalat; Hydroxyalkylcelluloseacetatsuccinate wie Hydroxypropylmethylcelluloseacetatsuccinat; Vinylacetatphthalate; Vinylacetatsuccinat; Celluloseacetattrimellitat, Polyvinylderivate wie Polyvinylacetatphthalat, Polyvinylalkoholphthalat, Polyvinylbutylatphthalat und Polyvinylacetoacetalphthalat; Zein; Schellack; oder Gemische davon.

11. Flüssige Zusammensetzung mit verlängerter Freisetzung nach Anspruch 9, wobei das pH-Wert-unabhängige Polymer aus der Gruppe ausgewählt ist, welche umfasst: cellulosische Polymere wie Ethylcellulose, Methylcellulose, Hydroxyethylcellulose, Hydroxypropylcellulose, Hydroxyethylmethylcellulose, Hydroxypropylmethylcellulose und Carboxymethylcellulose; Acrylcopolymere wie Methacrylsäure-Copolymere, *z*.. Eudragit® RS, Eudragit® RL, Eudragit® NE 30 D; Celluloseacetat; Polyethylenderivate, *z*. *B.* Polyethylenglycol und Polyethylenoxid; Polyvinylalkohol; Polyvinylacetat; Gummis, *z*. *B.* Guargummi, Johannisbrotkernmehl, Traganth, Carrageenan, Alginsäure, Akaziengummi, Gummiarabikum, Gellangummi und Xanthangummi; Triglyceride; Wachse, *z*. *B.* Compritol®, Lubritab® und Gelucires®; Lipide; Fettsäuren oder deren Salze/Derivate; ein Gemisch aus Polyvinylacetat und Polyvinylpyrrolidon, *z*. *B.* Kollidon® SR; oder Gemische davon.

12. Flüssige Zusammensetzung mit verlängerter Freisetzung nach Anspruch 1, wobei die Suspensionsgrundlage ferner einen oder mehrere pharmazeutisch annehmbare Hilfsstoffe umfasst, ausgewählt aus der Gruppe, umfassend: Suspendiermittel, Antiagglomerationsmittel, Netzmittel, Konservierungsmittel, Puffermittel, Aromamittel, Antioxidantien und Chelatbildner.

13. Flüssige Zusammensetzung mit verlängerter Freisetzung nach Anspruch 1, wobei die Zusammensetzung ferner Metformin in einer Form mit sofortiger Freisetzung beinhaltet.

14. Flüssige Zusammensetzung mit verlängerter Freisetzung nach Anspruch 1, wobei die Zusammensetzung ferner ein zusätzliches Antidiabetikum beinhaltet.

15. Flüssige Zusammensetzung mit verlängerter Freisetzung nach Anspruch 1 zur Benutzung in der Behandlung von Diabetes Typ II durch Verabreichung einmal oder zweimal täglich.

16. Flüssige Zusammensetzung mit verlängerter Freisetzung nach Anspruch 15, ferner umfassend ein oder mehrere Antidiabetika, ausgewählt aus der Gruppe, umfassend: Acarbose, Miglitol, Voglibose, Repaglinid, Nateglinid, Glibenclamid, Glimepirid, Glipizid, Gliclazid, Chloropropamid, Tolbutamid, Phenformin, Alogliptin, Sitagliptin, Linagliptin, Saxagliptin, Rosiglitazon, Pioglitazon, Troglitazon, Faraglitazar, Englitazon, Darglitazon, Isaglitazon, Zorglitazon, Liraglutid, Muraglitazar, Peliglitazar, Tesaglitazar, Canagliflozin, Dapagliflozin, Remogliflozin, Sergliflozin oder Gemische davon.

## Revendications

1. Composition liquide à libération prolongée de metformine comprenant ;
(i) Des noyaux de metformine recouverts d'un polymère contrôlant la libération ; et
(ii) une base de suspension ;
dans lequel la composition est une suspension ou une poudre reconstituée pour suspension **caractérisée en ce qu'**elle a *in-vitro* un profil de libération de dissolution tel que déterminé par l'appareil USP de type II à 100 tr/min, dans 1 000 ml de tampon phosphate avec un pH de 6,8 à 37°C comme suit :
- pas moins de 20% de metformine libérée après 1 heure,
- pas moins de 70% de metformine libérée après 4 heures,
- pas moins de 85% de metformine libérée après 8 heures, et
- pas moins de 90% de metformine libérée après 10 heures,
et dans lequel un profil de libération de dissolution *in-vitro* de la composition liquide à libération prolongée après stockage pendant au moins sept jours reste sensiblement similaire au profil de libération de dissolution *in vitro* initial.

2. Composition liquide à libération prolongée selon la revendication 1, dans laquelle la composition est **caractérisée en ce qu'**elle a un rapport d'osmolalité d'au moins environ 1.

3. Composition liquide à libération prolongée selon la revendication 1, dans laquelle la base de suspension comprend un agent osmotique.

4. Composition liquide à libération prolongée selon la revendication 1, dans laquelle la composition est une suspension en particulier une suspension reconstituée à partir d'une poudre pour suspension dont le taux de dissolution *in vitro* est mesuré après la constitution de la suspension.

5. Composition liquide à libération prolongée selon la revendication 1, dans laquelle la composition a un pH compris entre environ 4 et environ 10.

6. Composition liquide à libération prolongée selon la revendication 1, dans laquelle la metformine est superposée sur une particule inerte pour former le noyau.

7. Composition liquide à libération prolongée selon la revendication 6, dans laquelle la particule inerte est sélectionnée parmi un groupe comprenant d'un graine nonpareil, d'une sphère de cellulose microcristalline, d'une perle de phosphate de calcium dibasique, d'une perle de mannitol, une perle de silice, d'une pastille d'acide tartrique ou d'une pastille à base de cire.

8. Composition liquide à libération prolongée selon la revendication 3, dans laquelle l'agent osmotique est sélectionné parmi le groupe comprenant des glucides tels que le xylitol, le mannitol, le sorbitol, l'arabinose, le ribose, le xylose, le glucose, le fructose, le mannose, le galactose, le saccharose, le maltose, le lactose, le dextrose et le raffinose ; des sels solubles dans l'eau d'acides inorganiques tels que le chlorure de magnésium, le sulfate de magnésium, le sulfate de potassium, le chlorure de lithium, le chlorure de sodium, le chlorure de potassium, l'hydrogénophosphate de lithium, l'hydrogénophosphate de sodium, l'hydrogénophosphate de potassium, le dihydrogénophosphate de lithium, le dihydrogénophosphate de sodium, le dihydrogénophosphate de potassium et le phosphate de sodium tribasique ; des sels solubles dans l'eau d'acides organiques tels que l'acétate de sodium, l'acétate de potassium, le succinate de magnésium, le benzoate de sodium, le citrate de sodium et l'ascorbate de sodium ; des acides aminés solubles dans l'eau tels que la glycine, la leucine, l'alanine, la méthionine ; l'urée ou ses dérivés ; de propylène glycol ; de glycérine ; ou de leurs mélanges.

9. Composition liquide à libération prolongée selon la revendication 1, dans laquelle le polymère contrôlant la libération est choisi parmi le groupe comprenant un polymère dépendant du pH, un polymère indépendant du pH ou des mélanges de ceux-ci.

10. Composition liquide à libération prolongée selon la revendication 9, dans laquelle le polymère dépendant du pH est sélectionné parmi le groupe comprenant des copolymères acryliques tels que les copolymères d'acide méthacrylique et de méthacrylate de méthyle, *p*.*ex*., Eudragit® L 100 et Eudragit® S 100, copolymères d'acide méthacrylique et d'acrylate d'éthyle, *p.ex.*, Eudragit® L 100-55 et Eudragit® L 30 D-55, copolymères de méthacrylate de diméthylaminoéthyle et de méthacrylate de butyle et de méthacrylate de méthyle *p.ex.*, Eudragit® E 100, Eudragit® E PO, copolymères d'acrylate de méthyle et d'acide méthacrylique et d'acide méthacrylique et octylacrylate, styrène et copolymères d'acide acrylique, et copolymère éthylacrylate-acide méthacrylique ; phtalate d'acétate de cellulose ; succinates d'acétate de cellulose ; phtalates d'hydroxyalkylcellulose tels que phtalate d'hydroxypropylméthylcellulose ; succinates d'acétate d'hydroxyalkylcellulose tels que succinate d'acétate d'hydroxypropylméthylcellulose ; phtalates d'acétate de vinyle ; succinate d'acétate de vinyle ; trimelliate d'acétate de cellulose ; dérivés de polyvinyle tels que phtalate d'acétate de polyvinyle, phtalate d'alcool polyvinyle, phtalate de butylate polyvinyle et phtalate d'lacétoacétal de polyvinyle ; zéine ; laque en écaille ; ou leurs mélanges.

11. Composition liquide à libération prolongée selon la revendication 9, dans laquelle le polymère indépendant du pH est sélectionné parmi le groupe comprenant des polymères cellulosiques tels que éthylcellulose, méthylcellulose, hydroxyéthylcellulose, hydroxypropylcellulose, hydroxyéthylméthylcellulose, hydroxypropylméthylcellulose et carboxy méthylcellulose ; des copolymères acryliques tels que copolymères d'acide méthacrylique, *p.ex.*, Eudragit® RS, Eudragit® RL, Eudragit® NE 30 D ; acétate de cellulose ; dérivés de polyéthylène *p.ex.*, polyéthylène glycol et oxyde de polyéthylène ; alcool polyvinyle ; acétate polyvinyle ; gommes *p.ex*., gomme de guar, gomme de caroube, tragacanthe, carraghénane, acide alginique, gomme d'acacia, gomme arabique, gomme gellane et gomme xanthane ; triglycérides; cires, *p.ex.*, Compritol®, Lubritab® et Gelucires® ; lipides ; acides gras ou leurs sels/ dérivés ; un mélange d'acétate polyvinyle et de polyvinylpyrrolidone, *p.ex.*, Kollidon® SR ; ou mélanges de ceux-ci.

12. Composition liquide à libération prolongée selon la revendication 1, dans laquelle la base de suspension comprend en outre un ou plusieurs excipients pharmaceutiquement acceptables sélectionnés parmi le groupe comprenant des agents de suspension, des agents antiagglomérants, des agents mouillants, des conservateurs, des agents tampons, des agents aromatisants, des antioxydants, et des agents chélateurs.

13. Composition liquide à libération prolongée selon la revendication 1, dans laquelle la composition comprend en outre la metformine sous une forme à libération immédiate.

14. Composition liquide à libération prolongée selon la revendication 1, dans laquelle la composition comprend en outre un médicament antidiabétique supplémentaire.

15. Composition liquide à libération prolongée selon la revendication 1, pour utilisation dans le traitement de diabètes de type II par administration une ou deux fois par jour.

16. Composition liquide à libération prolongée selon la revendication 15, qui comprend en outre un ou plusieurs médicaments antidiabétiques sélectionnés parmi le groupe comprenant acarbose, miglitol, voglibose, repaglinide, natéglinide, glibenclamide, glimepride, glipizide, gliclazide, chloropropamide, tolbutamide, phénformine, aloglitine, sitagliptine, linagliptine, saxagliptine, rosiglitazone, pioglitazone, troglitazone, faraglitazar, anglitazone, darglitazone, isaglitazone, zorglitazone, liraglutide, muraglitazar, peliglitazar, tesaglitazar, canagliflozine, dapagliflozine, remogliflozine, sergliflozine, ou leurs mélanges.
